# EUROPÄISCHE PATENTSCHRIFT

(11) **EP 0 881 206 B1**
(45) Veröffentlichungstag und Bekanntmachung des Hinweises auf die Patenterteilung: **28.11.2001**
(21) Anmeldenummer: 98108692.9
(22) Anmeldetag: 13.05.1998
(51) Int. Cl.: C07C 45/38, C07C 45/67, C07C 47/21

(54) **Verfahren zur kontinuierlichen technischen Herstellung ungesättigter aliphatischer Aldehyde in einem Rohrbündelreaktor**
Process for the technical preparation of unsaturated aliphatic aldehydes in a tube bundle reactor
Procédé pour la préparation technique d'aldéhydes aliphatiques insaturés dans un réacteur à faisceau tubulaire

(30) Priorität: 28.05.1997 DE 19722567
(43) Veröffentlichungstag der Anmeldung: 02.12.1998
(73) Patentinhaber: BASF AKTIENGESELLSCHAFT, 67056 Ludwigshafen (DE)
(72) Erfinder: Aquila, Werner, Dr., 68309 Mannheim (DE); Fuchs, Hartwig, 67063 Ludwigshafen (DE); Wörz, Otto, Dr., 67159 Friedelsheim (DE); Ruppel, Wilhelm, Dr., 67227 Frankenthal (DE); Halbritter, Klaus, Dr., 69124 Heidelberg (DE)

(56) Entgegenhaltungen:
- EP-A- 0 263 385
- FR-A- 2 231 650
- FR-A- 2 386 509
- US-A- 3 284 170
- US-A- 4 097 535
- US-A- 5 149 884

## Beschreibung

Die Erfindung betrifft eine Verbesserung der kontinuierlichen technischen Herstellung von ungesättigten aliphatischen Aldehyden durch katalytisch beschleunigte oxigenierende Dehydrierung von ungesättigten aliphatischen Alkoholen in einem Rohrbündelreaktor, insbesondere eine Verbesserung bei der kontinuierlichen Herstellung von 3-Methyl-2-buten-1-al (Prenal) aus 3-Methyl-2-buten-1-ol (Prenol) und/oder 3-Methyl-3-buten-1-ol (Isoprenol).

Problematisch an dieser Umsetzung ist, daß sie stark exotherm ist, die Reaktionsgeschwindigkeit stark von der Reaktionstemperatur abhängig ist und daß die Reaktanten sowie die Reaktionsprodukte äußerst instabil sind.

Es ist aus der US-A 2 042 220 bekannt, daß man Isoprenol mit einem Überschuß an Sauerstoff bei 360 bis 550°C in Gegenwart von Metallkatalysatoren, z.B. Kupfer- und Silberkatalysatoren, zu 3-Methyl3-buten-1-al (Isoprenal) oxidieren kann. Die Katalysatoren können Legierungen, Metallverbindungen oder elementares Metall sein. Bevorzugt sind aktivierte Katalysatoren; als Aktivierungsoperationen werden eine Oberflächenamalgamierung des Metalls und anschließendes Erhitzen der Metalloberfläche angegeben. Die Herstellung von Kupfer- und Silberkatalysatoren in den Beispielen besteht in der Reduktion von Kupferoxidpartikeln bei 300°C unter Wasserstoffatmosphäre oder in Amalgamierung und Erhitzen von Silberdrahtnetzen. Gemäß DE-B-20 41 976 werden bei diesem aus US-A 2 042 220 bekannten Verfahren erhebliche Mengen an unerwünschten Nebenprodukten gebildet.

In DE-A-25 17 859 wird die Dehydrierung ungesättigter Alkohole an einem Kupferkatalysator, der eine spezifische Oberfläche von 0,01 bis 1,5 m²/g hat, im wesentlichen in Abwesenheit von Sauerstoff bei 150 bis 300°C beschrieben. Im Falle von α,β-ungesättigten Alkoholen als Ausgangsstoffen werden β,γ-ungesättigte Aldehyde und gesättigte Aldehyde als Nebenprodukte gebildet; die Selektivität für α,β-ungesättigte Aldehyde ist gering (vgl. Seite 2, letzter Absatz). Solche Gemische müssen in aufwendigen Trennoperationen in ihre Komponenten zerlegt werden.

In der DE-B-20 20 865 und der DE-B-20 41 976 wird die Dehydrierung von β,γ-ungesättigten Alkoholen bzw. α,β-ungesättigten Alkoholen zu α,β-ungesättigten Aldehyden beschrieben. Als Dehydrierungskatalysatoren werden auch Mischkatalysatoren, z.B. solche aus Kupfer und Silber genannt. Nachteilig ist jedoch, daß man erhebliche Mengen an nukleophilen Substanzen zusetzen muß. Im Falle der Umsetzung von 3-Methyl-3-buten-1-ol werden gute Ergebnisse nur bei unvollständigen Umsätzen erhalten, was nach der Lehre der DE-B-22 43 810 zu Schwierigkeiten bei der Abtrennung des unumgesetzten Ausgangsstoffes führt.

Dehydriert man Isoprenol nach dem Verfahren der DE-B-25 17 859 ohne Zusatz von Sauerstoff an metallischem Kupfer, so entstehen erhebliche Mengen an Isovaleraldehyd und die Aktivität der Katalysatoren fällt innerhalb weniger Tage rasch ab, so daß häufig regeneriert werden muß.

In der FR-A-2 231 650 wird die Herstellung von Aldehyden und Ketonen aus den entsprechenden Alkoholen durch Luftoxidation bei 250 bis 600°C in Gegenwart eines Gold-Katalysators beschrieben. Der Vorteil des Gold-Katalysators liegt in der höheren Selektivität im Vergleich zu Kupfer und Silber-Katalysatoren, so daß die Nebenprodukt-Bildung verringert wird. Nachteilig bei diesem Verfahren sind die hohen Katalysatorkosten, da ein massiver Gold-Kontakt Verwendung findet.

In den Patenten DE-B-27 15 209 und EP-B-55 354 wird die oxidative Dehydrierung von 3-Alkyl-buten-1-olen an Katalysatoren, die aus Schichten von Silber- und/oder Kupferkristallen bestehen, unter Zusatz von molekularem Sauerstoff beschrieben. Die Sauerstoffmengen liegen im Bereich von 0,3 bis 0,7 mol, bezogen auf den Einsatzstoff. Nachteilig bei diesem Verfahren ist, daß hohe Katalysatorkosten durch Verwendung von massivem Silber auftreten und gute Selektivitäten nur erzielt werden können, wenn definierte Katalysatorkorngrößen bzw. -korngrößenverteilungen in einem Schichtenaufbau, unter Umständen sogar bestimmte Mischungen von Schichten aus Kupfer- und Silberkristallen eingesetzt werden. Dies bedeutet sowohl eine aufwendige Füllung des Reaktors als auch eine aufwendige Katalysatorrückgewinnung. Zudem tritt bei den dabei angewandten hohen Reaktionstemperaturen Sinterung der Metallkristalle auf, was zu Druckanstieg und geringen Standzeiten führt.

In der JP-A-60/246340 wird die Gasphasenoxidation von Prenol zu Prenal bei 300 bis 600°C in Gegenwart von Sauerstoff und eines Trägerkatalysators beschrieben. Der Trägerkatalysator ist in komplizierter Weise durch Tränken des Trägers mit wäßrigen Lösungen von AgNO₂, Cu(NO₃)₂ x 3 H₂O und Mg(NO₃)₂ x 6 H₂O, Trocknen, Calcinieren in einem bestimmten Temperaturbereich sowie Aktivieren unter Wasserstoffatmosphäre herzustellen. Der Katalysator liefert zwar eine gute Selektivität von 96,6 %, jedoch nur unter Inkaufnahme von geringem Umsatz, so daß er für technische Zwecke kaum in Betracht kommt.

In der JP-A-58/059 933 wird die Herstellung von Aldehyden und Ketonen durch oxidative Dehydrierung von Alkoholen in Gegenwart eines Silberkatalysators, der zusätzlich Phosphor enthält, beschrieben. Um die Selektivität der Umsetzung aufrechtzuerhalten, wird in den Alkoholstrom zusätzlich eine Phosphorverbindung eingebracht, so daß man eine Verunreinigung des Produktes in Kauf nehmen muß. In Anbetracht der angestrebten Verwendung der Aldehyde für Riechstoffe und Vitamine ist der Zusatz einer phosphororganischen Verbindung evident nachteilig.

Gemäß EP 244 632 B1 gelingt die kontinuierlich oxidierende Dehydrierung selbst ungesättigter aliphatischer Alkohole zu den entsprechenden Aldehyden recht vorteilhaft, wenn man die Umsetzung in der Gasphase bei 300 bis 600°C an einem geeigneten Katalysator durchführt, der sich in zwischen Rohrböden angeordneten kurzen und dünnen Reaktionsrohren befindet, die von einem fluiden Wärmeträger in seitlicher Richtung umströmt werden.

Sehr vorteilhaft gestaltete sich das Verfahren gemäß EP 244 632, wenn man das aus dem Rohrbündelreaktor austretende dampfförmige Gasgemisch kurz nach der Berührung mit dem Katalysator mit Wasser und/oder einem Wasser und unumgesetzte Alkohole enthaltenden kondensierten Reaktionsgemisch bei Temperaturen von -20 bis +50°C in Berührung bringt, aus dem erhaltenen Kondensat die Aldehyde abtrennt, wie es in EP 55 354 A1 beschrieben ist und die unumgesetzten Alkohole in den Prozeß zurückführt.

Nachteilig an dieser an sich sehr vorteilhaften Verfahrenskombination ist, daß bei der kontinuierlichen Durchführung des Verfahrens trotz häufigen Abbrennens von Ruß und anderen Verkrustungen auf dem Katalysator nach einigen Wochen sowohl der Umsatz als auch die Selektivität stark abfallen (siehe Vergleichsbeispiel), so daß der Katalysator ausgewechselt werden muß. Beim Öffnen eines solchen Reaktors zeigte sich, daß die Reaktionsrohre zum überwiegenden Teil total verstopft waren. Die Pfropfen in den einzelnen Rohren waren so hart, daß die betroffenen Rohre mit einer Schlagbohrmaschine aufgebohrt werden mußten, was technisch sehr aufwendig ist und zu Beschädigungen der Rohre führen kann. Das langsame Verstopfen der einzelnen Rohre ließ sich weder durch Vorschalten einer geordneten Packung aus Metallgewebe (Sulzer BX) als Vorfilter noch durch Überschichten der Katalysatorschicht mit inerten Partikeln, wie Glas- oder Porzellankugeln als Filter verhindern.

Ein weiterer Nachteil dieses Verfahren ist, daß der Bau von Rohrbündelreaktoren mit sehr kurzen Reaktionsrohren für solche Rohrbündelreaktoren, in denen mit hohen Kapazitäten, d.h. mit einer sehr großen Anzahl von Reaktionsrohren, gearbeitet werden soll, fertigungstechnisch Schwierigkeiten bereitet.

Es war daher die Aufgabe der Erfindung, das oben beschriebene Verfahren gemäß EP 244 632 B1 zur kontinuierlichen technischen Herstellung ungesättigter aliphatischer Aldehyde durch oxigenierende Dehydrierung der entsprechenden Alkohole mit einem Sauerstoff-enthaltenden Gas an einem Trägerkatalysator bestehend aus Kupfer, Silber und/oder Gold auf einem inerten Träger in einem Rohrbündelreaktor, schnelles Abkühlen der Reaktionsgase und Abtrennen der Aldehyde aus dem erhaltenen Kondensat so zu verbessern, daß die Nachteile des Standes der Technik nicht auftreten, d.h., daß man das kontinuierliche Verfahren über lange Zeiträume, möglichst über mehrere Jahre, ohne Abschalten der Anlage und ohne aufwendiges Ausbohren der Reaktionsrohre in einem problemlos herstellbaren Reaktor betreiben kann.

Gegenstand der Erfindung ist nun ein Verfahren zur kontinuierlichen Herstellung von ungesättigten aliphatischen Aldehyden der allgemeinen Formel I 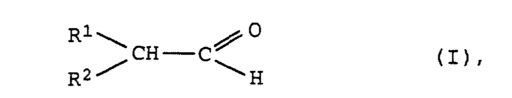 in der R¹ für Wasserstoff steht und R² den Rest 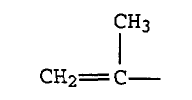 (Isoprenal) bedeutet oder R¹ und R² zusammen den Rest 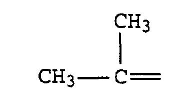 (Prenal) bedeuten, durch oxigenierende Dehydrierung von einem der beiden oder einem Gemisch aus beiden 3-Alkyl-buten-1-olen der allgemeinen Formel II 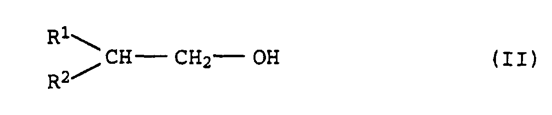 (Prenol und/oder Isoprenol) mit einem Sauerstoff-enthaltenden Gas an einem Trägerkatalysator bestehend aus Kupfer, Silber und/oder Gold auf einem inerten Träger in einem Rohrbündelreaktor, schnelles Abkühlen der Reaktionsgase und Abtrennen der Aldehyde aus dem erhaltenen Kondensat, das dadurch gekennzeichnet ist, daß man
a) den Dampf von einem oder beiden der 3-Alkyl-buten-1-ole der allgemeinen Formel II verdampft,
b) den Alkoholdampf (1) mit einem Sauerstoff-enthaltenden Gas (2) versetzt,
c) den erhaltenen Sauerstoff-enthaltenden Alkoholdampf (1+2) zunächst bei Temperaturen oberhalb des Taupunktes des Alkohols aber unterhalb der Starttemperatur der Reaktion durch eine mindestens 0,5 cm dicke Schicht, vorzugsweise eine 0,6 bis 5 cm dicke Schicht (3a), eines der oben genannten Trägerkatalysatoren, die vorzugsweise den Querschnitt des gesamten Reaktors einnimmt, leitet, und erst danach
d) den Sauerstoff-enthaltenden Alkoholdampf in einer der gewünschten Kapazität entsprechenden Anzahl von parallel angeordneten und von mit einem fluiden Wärmeträger (4) umströmten und mit einem der genannten Trägerkatalysator gefüllten Reaktionsrohren (3b) mit einem Innendurchmesser D von etwa 0,5 bis 3 cm, vorzugsweise 1 bis 2 cm, und einer Länge von mindestens 5 cm, vorzugsweise 35 bis 60 cm, bei Temperaturen von 300 bis 600°C zu dem entsprechenden Aldehyd reagieren läßt.

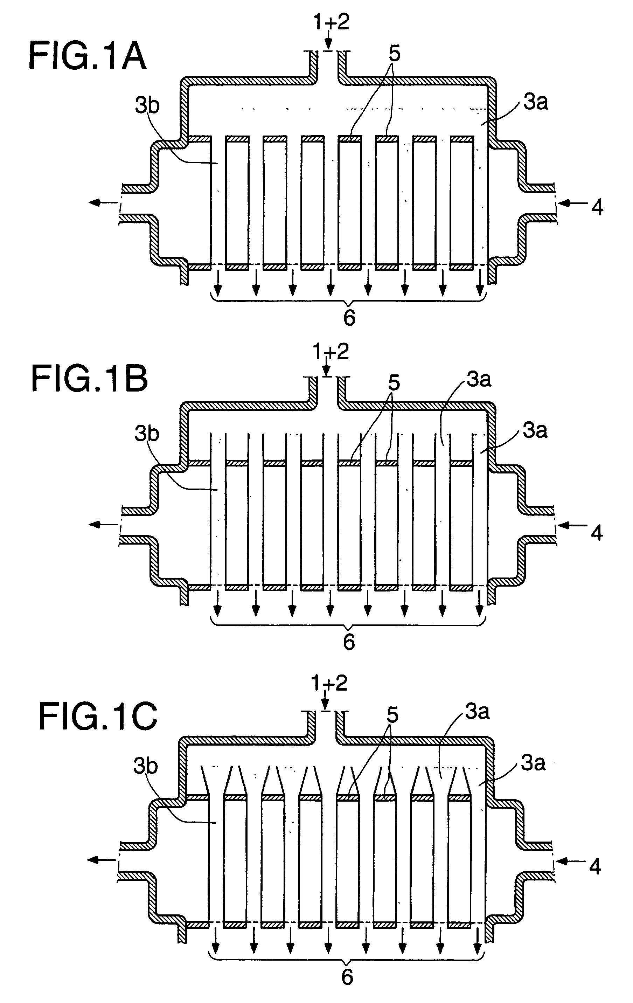

Das Verfahren ist in drei seiner vorteilhaften Ausführungsformen schematisch in den Figuren 1a, 1b und 1c dargestellt.

Hierin bedeuten:
- 1: den Alkoholdampf,
- 2: das Sauerstoff enthaltende Gas,
- 3a: die mindestens 0,5 cm dicke Schicht eines der Trägerkatalysatoren, die vorzugsweise den Querschnitt des gesamten Reaktors einnimmt (Fig. 1a),
- 3b: die mit einem Trägerkatalysator gefüllten und mit einem fluiden Wärmeträger umströmten Reaktionsrohre,
- 4: den fluiden Wärmeüberträger,
- 5: den Rohrboden (auch Rohrspiegel genannt)
und
- 6: das den erhaltenen Aldehyd und unumgesetzten Alkohol enthaltende dampfförmige Reaktionsgemisch.

Mit Vorteil gelingt das erfindungsgemäße Verfahren, wenn man als Trägerkatalysator einen Trägerkatalysator bestehend aus metallischem Silber auf einem inerten Träger verwendet.

In der Praxis am besten zu handhaben ist es, wenn man in den Reaktionsschritten c) und d) als Trägerkatalysator den gleichen Silber-Trägerkatalysator verwendet und die Katalysatoren der Reaktionsschritte c) und d) in unmittelbarem Kontakt stehen.

Mit besonderem Vorteil gelingt das erfindungsgemäße Verfahren, wenn man in den Reaktionsschritten c) und d) einen Silber-Trägerkatalysator verwendet, der aus Kugeln eines inerten Trägermaterials bestehen, die mit 0,1 bis 20 Gew.-%, bezogen auf die Menge des Trägers, einer Schicht aus metallischem Silber in Form einer glatten, abriebfesten Schale beschichtet sind und wobei für den größten Durchmesser d der beschichteten Trägerkatalysatorkugeln ein Verhältnis zu dem Innendurchmesser D der Reaktionsrohre von d/D = 0,05 bis 0,3, vorzugsweise 0,1 bis 0,2, gilt.

Wie dem Beispiel 1 zu entnehmen ist, konnte nach Anwenden der erfindungsgemäßen Maßnahmen der Rohrbündelreaktor mehr als 3 Jahre ohne Kapazitätsverlust und ohne Katalysatorwechsel für die kontinuierlich technische Herstellung von Prenal verwendet werden.

Das aufwendige Ausbohren der Reaktionsrohre entfällt, der Katalysator kann einfach durch Arbeiten mit einem Staubsauger ausgetragen werden. Es war sehr überraschend, daß durch so einfache Maßnahmen solch ein vorteilhafter Effekt zu erzielen war.

Ein weiterer Vorteil des erfindungsgemäßen Verfahrens ist, daß man den Reaktor mit höherer Belastung fahren kann.

Für die Herstellung von Aldehyden durch katalytische Oxidation von Alkoholen an Silberkatalysatoren ist charakteristisch, daß mit steigendem Umsatz die Selektivität sinkt. Aus US 4 097 535 war schon bekannt, daß man z.B. bei der Oxidation von Octanol an Silberkatalysatoren den Umsatz von 77 % auf 91 % steigern kann, ohne daß die Selektivität abfällt, wenn man das Gemisch aus dem Alkohol und dem Sauerstoff-enthaltenden Gas zunächst über eine Silber-Trägerkatalysator enthaltende Vor-Reaktorzone und erst dann in die Reaktorzone mit einer Temperatur von 300 bis 600°C leitet, jedoch zeigen die Beispiele deutlich, daß das hier erstrebte Ziel, nämlich die Steigerung des Umsatzes, verständlicherweise nur erreicht werden kann, wenn in der Vor-Reaktorzone, die durch externe Heizquellen temperiert wird, Temperaturen oberhalb der Starttemperatur der Reaktion herrschen.

Demgegenüber sind die Temperaturen in der Schicht des Trägerkatalysators gemäß Verfahrensschritt c) des erfindungsgemäßen Verfahrens unterhalb der Starttemperatur der Reaktion. Der Umsatz ist dementsprechend auch nicht größer als ohne Anwendung der erfindungsgemäßen Maßnahmen. Das kontinuierliche technische Verfahren arbeitet mit Vorteil bei Umsätzen von 50 bis 60 % und unter Rückführung des nicht umgesetzten Alkohols. Vermutlich führen geringste Mengen an Nebenprodukten in dem durch Rückführung von unumgesetztem Alkohol ergänzten Ausgangsalkohol zu den extremen Verstopfungen in den Reaktionsrohren. Überraschenderweise werden diese den Katalysator zerstörenden Reaktionen dadurch vermieden, daß man den Sauerstoff enthaltenden Alkoholdampf zunächst bei Temperaturen unterhalb der Starttemperatur über einen geeigneten Trägerkatalysator, vorzugsweise einen Silber-Trägerkatalysator, insbesondere über den gleichen Silber-Trägerkatalysator wie in Reaktionsschritt d) leitet. Bei der Herstellung von Prenal ergibt sich eine besonders vorteilhafte Ausführungsform dadurch, daß man einfach den aus einem Verdampfer kommenden Alkoholdampf mit auf Temperaturen von 140 bis 160°C erhitzter Luft mischt, und das so erhaltene 120 bis 130°C heiße Alkohol-Luft-Gemisch bei diesen Temperaturen in die nicht anderweitig beheizte Trägerkatalysatorschicht gemäß Verfahrensschritt c) leitet.

Die Dicke dieser Katalysatorschicht ist einerseits abhängig von der Reaktorkonstruktion, andererseits von dem Durchmesser der Katalysatorpartikel. Im allgemeinen benötigt man bei größeren Reaktoren eine etwas dickere Schicht, um sicherzustellen, daß nicht einzelne Reaktionsrohre nur unzureichend geschützt sind und dadurch verstopfen. Die Schicht muß andererseits mindestens die Dicke von 2 mal dem Korndurchmesser der Katalysatorpartikel aufweisen. So ergeben sich Schichtdicken von mindestens 0,5 cm, vorzugsweise 0,6 bis 5 cm, insbesondere 1 bis 3 cm.

Überraschenderweise hat sich ergeben, daß bei Anwenden des erfindungsgemäßen Reaktionsschrittes c) auch solche Reaktionsrohre im Rohrbündelreaktor verwendet werden können, die länger sind als die in EP 244 632 beschriebenen relativ kurzen Rohre, was sich sowohl im Hinblick auf den Bau der Reaktoren als auch hinsichtlich der Standzeit des Katalysators positiv auswirkt.

Als Trägerkatalysatoren für das erfindungsgemäße Verfahren kommen Partikel in Betracht, die metallisches Kupfer, Silber und/oder Gold, vorzugsweise Silber, auf einem inerten Trägermaterial enthalten.

Gegebenenfalls kann der Katalysator auch mit einem inerten, nicht mit aktiver Masse beschichteten Material verdünnt werden. Als inerte Materialien, die auch als Trägermaterial geeignet sind, kommen keramische Massen, wie Aluminiumoxid, Siliciumdioxid, Magnesiumoxid, Siliciumcarbid oder vor allem Steatit in Frage. Eine Kontaktschicht sollte aber mindestens 10 % an Teilchen mit aktiver Masse enthalten.

Als inerte Formkörper für den Katalysator eignen sich in erster Linie Kugeln sowie daneben auch sonstige Körper wie Ellipsoide, Zylinder oder Ringe. Der Durchmesser d der Kugeln, bzw. der größte Durchmesser der sonstigen Körper, kann im Bereich von 0,1 bis 1,5 cm Durchmesser liegen, wobei sich die Durchmesser nach dem inneren Durchmesser der Rohrbündelrohre richten.

Die Katalysatorteilchen werden beispielsweise auf ein Netz aus Silber oder Edelstahl in den üblicherweise vertikal aufgestellten Reaktor geschichtet.

Das als Katalysator aktive Metall wird vorzugsweise durch Flammspritzen auf das Inertmaterial aufgebracht, jedoch kommen auch andere Methoden z.B. Tränken oder Plasmaspritzen in Betracht, sofern dabei eine abriebfeste Schale entsteht, die im übrigen möglichst glatt sein sollte.

Der Katalysator ist einfach herzustellen und erlaubt besonders im Falle von Kugeln eine einfache Befüllung des Reaktors. Ein weiterer Vorteil der regelmäßigen Form des Katalysators ist, daß ohne weitere Maßnahmen eine geordnete dichteste Packung im Reaktor erzielt wird und bei Rohrbündelreaktoren jedes Einzelrohr des Bündels aufgrund der gleichmäßigen Schüttung einen sehr ähnlichen Druckverlust zeigt. Der in den vielen Rohren eines Rohrbündelreaktors auftretende gleiche Druckverlust führt zu gleicher Anströmung der einzelnen Rohre und dadurch offenbar zu einer wesentlichen Verbesserung der Selektivität der Umsetzung. Im Verlauf der Reaktion werden einzelne Rohre nicht stärker belastet, so daß die Standzeit des Katalysators unter den erfindungsgemäßen Bedingungen sehr hoch ist und in der Praxis bei mehreren Jahren liegt.

Als oxidierendes Agens lassen sich sowohl reiner Sauerstoff als auch freien Sauerstoff enthaltende Gase, insbesondere Luft, verwenden. Der Sauerstoff und der Alkohol werden zweckmäßig im Molverhältnis von 0,1 bis 0,8, insbesondere von 0,2 bis 0,4 Mol Sauerstoff je Mol Alkohol angewandt.

Je nach gewünschter Kapazität des Reaktors verwendet man einen Rohrbündelreaktor mit 100 bis 10000 Rohren einer Rohrlänge von mindestens 5, vorzugsweise 10 bis 60 cm, insbesondere 35 bis 60 cm. Für Versuchszwecke genügt die Verwendung eines Rohres.

Die Reaktion wird im allgemeinen bei Drucken zwischen 0,8 und 2 bar, vorzugsweise bei Normaldruck, kontinuierlich durchgeführt.

Zweckmäßig belastet man den Katalysator mit 0,5 bis 7 t, insbesondere 1 bis 5 t Alkohol pro m² Katalysatorquerschnitt und Stunde.

Das Reaktionsgemisch arbeitet man in an sich bekannter Weise auf. Beispielsweise absorbiert man die heißen Reaktionsgase unmittelbar nach Austritt aus dem Reaktor mit einem Lösungsmittel wie Wasser oder vorzugsweise im kondensierten Produktgemisch.

Die Verweilzeit des Gasgemisches im Reaktionsrohr beträgt 0,0005 bis 1, vorzugsweise 0,001 bis 0,05 Sekunden.

Als Ausgangsverbindungen kommen die Methylbutenole der allgemeinen Formel II 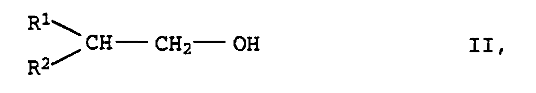 in denen R¹ und R² die oben angegebene Bedeutung haben, in Betracht. Die Methylbutenole sind bekannte Verbindungen und nach bekannten Verfahren erhältlich.

Für die Herstellung von Prenal ergibt sich eine besonders bevorzugte Ausführungsform des erfindungsgemäßen Verfahrens, die dadurch gekennzeichnet ist, daß man
a) Prenol und/oder Isoprenol verdampft,
b) den Prenol- und/oder Isoprenol-Dampf mit einem Sauerstoff-enthaltenden Gas versetzt,
c) den erhaltenen Sauerstoff-enthaltenden Dampf aus Prenol- und/ oder Isoprenol bei Temperaturen kurz oberhalb des Taupunktes durch eine 0,5 bis 4 cm dicke Silber-Trägerkatalysator-Schicht leitet, die vorzugsweise den Querschnitt des gesamten Reaktors einnimmt, danach
d) den Sauerstoff-enthaltenden Dampf aus Prenol- und/oder Isoprenol in einer der gewünschten Kapazität entsprechenden Anzahl von mit einem Silber-Trägerkatalysator gefüllten Reaktionsrohren mit einem Innendurchmesser von 1 bis 2 cm und einer Länge von 35 bis 60 cm zu einem Gemisch aus Prenal und Isoprenal reagieren läßt und
e) das in dem erhaltenenen Gemisch aus Prenal und Isoprenal enthaltene Isoprenal in an sich bekannter Weise zu Prenal isomerisiert.

Mit Hilfe des erfindungsgemäßen Verfahrens können die als Zwischenprodukte für die Herstellung von Riechstoffen, Vitaminen und Carotinoiden begehrten α,β-ungesättigten Aldehyde, insbesondere das begehrte Prenal, in vorteilhaft herstellbaren Rohrbündelreaktoren mit Katalysatorstandzeiten von mehreren Jahren in guten Ausbeuten hergestellt werden.

### Beispiel 1

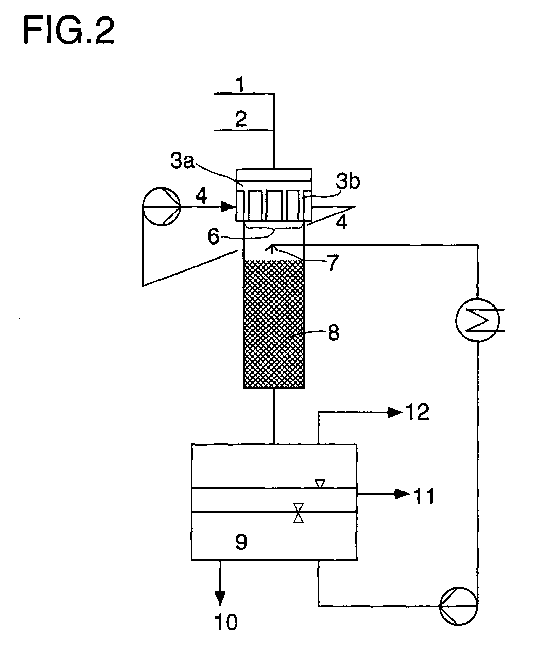

Es wurde eine technische Anlage verwendet, wie sie in Figur 2 schematisch dargestellt ist.

Rohalkohol, enthaltend ca. 70 bis 80 Gew.-% Isoprenol, wurde verdampft und der Dampf (1) in einer Mischstrecke mit der auf 140 bis 160°C erwärmten Luft (2) vermischt und dieses 125 bis 130°C heiße Gemisch zunächst durch die auf dem Rohrboden liegende Silber-Trägerkatalysatorschicht (3a) und dann in die mit dem Silber-Trägerkatalysator gefüllten und von der zum Starten der Reaktion und zur Reaktionswärmeabfuhr dienenden etwa 360°C heißen Salzschmelze (4) umströmten Reaktorrohre (3b) geleitet. Als Silber-Katalysator wurden die gleichen mit metallischem Silber beschichteten Stalaktitkügelchen verwendet, wie sie im Vergleichsbeispiel beschrieben werden.

Die bei der Umsetzung erhaltenenen heißen Reaktionsgase (6) wurden in einem Quench (7) mit anschließender Kolonne (8) der mit der Wasserphase (9) des Produktaustrages beschickt wurde, abgekühlt. Über die Leitungen (11) bzw. (10) wurden die beiden flüssigen Phasen des Reaktoraustrages zur Aufarbeitung bzw. zum Quenchen entnommen; über die Leitung (12) wurden die gasförmigen Anteile entsorgt.

Der oben beschriebene technische Reaktor wurde 1167 Tage ohne Katalysatorwechsel mit Belastungen zwischen 120 g und 360 g Rohalkohol betrieben.

Hierbei wurde der Betrieb wöchentlich einmal für einige Stunden unterbrochen, um Ruß und andere organische Ablagerungen auf dem Katalysator abzubrennen.

Am 1165. Tag wurde eine Bilanzierung analog dem Vergleichsbeispiel 1a durchgeführt. Dem Reaktor wurden pro Stunde (h) und Reaktionsrohr ein Gemisch aus 350,6 g Rohalkoholdampf mit einem Gehalt von 74,2 % 3-Methyl-3-buten-1-ol und 50,9 l (Nl) Luft mit einer Temperatur des Gemisches von 127°C zugeführt. Im Reaktoraustrag wurden nach Isomerisierung 122,0 g 3-Methyl-2-buten-1-al und 137,0 g 3-Methyl-3-buten-1-ol gefunden entsprechend einem Umsatz von 52,1 % und einer Selektivität von 91,2 %.

### Beispiel 2

In der in Figur 2 schematisch dargestellten und in Beispiel 1 näher beschriebenen technischen Anlage wurden 240,2 g Rohalkohol (1) (enthaltend 64,0 Gew.-% 3-Methyl-3-buten-1-ol und 12,5 % Gew.-% 3-Methyl-2-buten-1-ol) pro h und Reaktionsrohr verdampft und mit 37,1 l (Nl) erwärmter Luft (2) pro h und Reaktionsrohr vermischt. Das Dampf-Luft-Gemisch wurde mit einer Temperatur von 125°C zunächst durch die auf dem Rohrboden liegende Silber-Trägerkatalysator-Schicht (3a) und dann in die mit dem gleichen Silber-Katalysator gefüllten und von der zum Starten der Reaktion und zur Wärmeabführung dienenden Salzschmelze (4) umströmten Reaktionsrohre geleitet. Als Silber-Trägerkatalysator wurden die gleichen mit metallischem Silber beschichteten Stalaktitkügelchen verwendet, wie sie im Vergleichsbeispiel beschrieben wurden.

Nach Isomerisierung des Rohaustrags erhielt man 88,1 g 3-Methyl-2-buten-1-al, 53,7 g 3-Methyl-3-buten-1-ol und 10,5 g 3-Methyl-2-buten-1-ol.

Der Umsatz bezogen auf beide Alkohole betrug 54,3 % bei einer Selektivität von 90,4 %.

### Beispiel 3

Feststellung der Wirkung eines Silber-Trägerkatalysators bei Temperaturen von 250°C hinsichtlich der Bildung von 3-Methyl-2-buten-1-al

Für diesen Versuch wurde eine Laborapparatur mit einem Rohr von einem Innendurchmesser von 12 mm und einer Länge von 400 mm verwendet.

Dem Reaktionsrohr wurde eine 130°C heiße Mischung aus dem Dampf von 90 g reinem 3-Methyl-3-buten-1-ol und Luft (Gewichtsverhältnis von 3-Methyl-3-buten-1-ol zu Luft = 1:0,3) zugeführt, wobei der Silber-Trägerkatalysator im Reaktionsrohr auf eine Temperatur von 250°C eingestellt wurde. Im Reaktoraustrag konnte kein Prenal nachgewiesen werden, d.h. der Umsatz betrug 0 %.

Dieses Beispiel zeigt, daß bei Temperaturen von 250°C und damit Temperaturen, wie sie in der auf dem Rohrboden liegenden Silber-Trägerkatalysatorschicht maximal auftreten könnten, noch kein Umsatz zu Prenal erfolgt.

### Vergleichsbeispiel

### Herstellung von 3-Methyl-2-buten-1-al

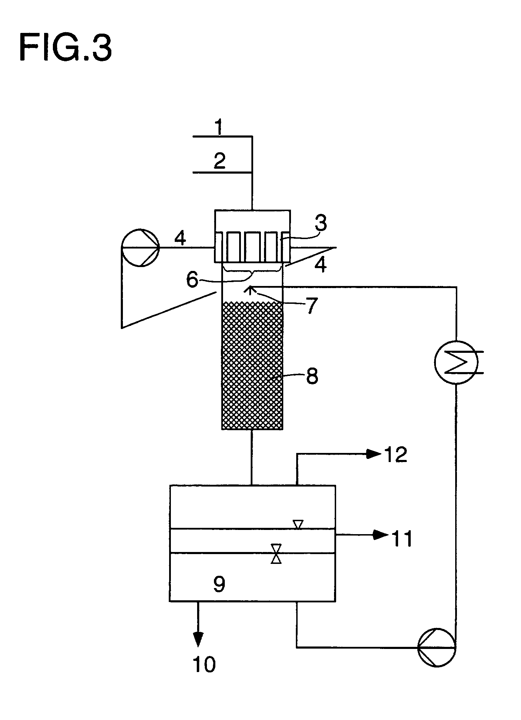

Es wurde eine technische Anlage verwendet, wie sie schematisch in Figur 3 dargestellt ist.

Ein Gemisch aus Prenol und Isoprenol wurde verdampft, der Dampf (1) in einer Mischstrecke mit der erwärmten Luft (2) vermischt und dieses Gemisch in die mit einem Silberkatalysator gefüllten Rohrbündelreaktorrohre (3) geleitet. Der Katalysator bestand aus einer abriebfesten Schicht von etwa 4 Gew.-% metallischem Silber auf Stalaktitkügelchen vom Durchmesser 0,2 bis 0,25 cm. Die Reaktorrohre waren von einer Salzschmelze (4) zum Starten der Reaktion und zur Wärmeabfuhr umströmt.

Die vereinigten heißen Reaktionsgase (6) wurden in einem Quench (7) mit anschließender Kolonne (8) der mit der Wasserphase (9) des Produktaustrages beschickt wurde, abgekühlt. Über die Leitungen (11) bzw. (10) wurden die beiden flüssigen Phasen des Reaktoraustrages zur Aufarbeitung bzw. zum Quenchen entnommen; über die Leitung (12) wurden die gasförmigen Anteile entsorgt.
a) Dem Reaktor wurden pro Stunde und Reaktionsrohr (12 mm 0̸) ein Gemisch aus 248 g Rohalkohol-Dampf und 34,4 l (Nl) Luft und einer Mischungstemperatur von 126°C zugeführt.
   Der Gehalt an 3-Methyl-3-buten-1-ol im Rohalkohol betrug 71,43 %. Die Temperatur der Salzschmelze um die Rohre wurde bei 360°C gehalten.
   Nach Isomerisierung des Quenchaustrags erhielt man 82,6 g Prenal (3-Methyl-2-buten-1-al )und 85,1 g nichtumgesetzten Alkohol entsprechend einem Umsatz von 52 % und einer Selektivität von 91,8 % der Theorie.
b) Dem technischen Reaktor wurden pro Stunde und Einzelrohr ein Gemisch aus 354,7 g Rohalkohol-Dampf mit einem Gehalt von 71,43 % 3-Methyl-3-buten-1-ol und 69,6 l (Nl) Luft zugeführt.
   Das Alkohol-Luft-Gemisch wies eine Temperatur von 130°C auf. Die Salzbadtemperatur wurde auf 360°C eingeregelt.
   Nach Isomerisierung des Rohaustrags erhielt man 165,2 g 3-Methyl-2-buten-1-al und 167,5 g 3-Methyl-3-buten-1-ol entsprechend einem Umsatz von 52,8 % und einer Selektivität von 90,4 % der Theorie.
c) Der oben beschriebene Reaktor wurde mit einem Gemisch aus Eduktdampf und Luft wie oben unter a) bzw. b) beschrieben 95 Tage betrieben. Hierbei wurde der Betrieb wöchentlich einmal für einige Stunden unterbrochen, um Ruß und andere organische Ablagerungen auf dem Katalysator abzubrennen. Nach jeweils 95 Tagen fielen Umsatz und Selektivität stark ab. Die Belastung pro Reaktionsrohr mußte auf 49 g Rohalkohol zurückgenommen werden.
   Nach Isomerisierung des Quenchaustrages erhielt man 8,9 g 3-Methyl-2-buten-1-al und 21 g 3-Methyl-3-buten-1-ol entsprechend einem Umsatz von nur noch 40 % und einer Selektivität von nur 65 % der Theorie.

## Patentansprüche

1. Verfahren zur kontinuierlichen technischen Herstellung von Aldehyden der allgemeinen Formel I 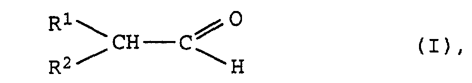 in der R¹ für Wasserstoff steht und R² den Rest 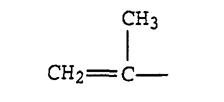 bedeutet (Isoprenal) oder R¹ und R² zusammen den Rest 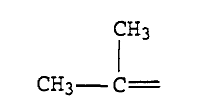 bedeuten (Prenal),
durch oxigenierende Dehydrierung der entsprechenden Alkohole mit einem Sauerstoff-enthaltenden Gas an einem Trägerkatalysator bestehend aus Kupfer, Silber und/oder Gold auf einem inerten Träger in einem Rohrbündelreaktor, schnelles Abkühlen der Reaktionsgase und Abtrennen der Aldehyde aus dem erhaltenen Kondensat unter Rückführen der nichtumgesetzten Alkohole, **dadurch gekennzeichnet, daß** man
a) 3-Alkyl-buten-1-ole der allgemeinen Formel II 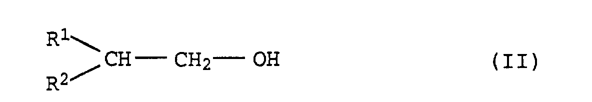 verdampft,
b) den Alkoholdampf mit einem Sauerstoff-enthaltenden Gas versetzt,
c) den erhaltenen Sauerstoff enthaltenden Alkoholdampf zunächst bei Temperaturen oberhalb des Taupunktes des Alkohols aber unterhalb der Starttemperatur der Reaktion durch eine mindestens 0,5 cm dicke Schicht eines der oben genannten Trägerkatalysatoren leitet und erst danach
d) den Sauerstoff enthaltenden Alkoholdampf in einer der gewünschten Kapazität entsprechenden Anzahl von parallel angeordneten und von mit einem fluiden Wärmeträger umströmten und mit dem Trägerkatalysator gefüllten Reaktionsrohren mit einem Innendurchmesser D von etwa 0,5 bis 3 cm und einer Länge von mindestens 5 cm bei Temperaturen von 300 bis 600°C zu dem entsprechenden Aldehyd reagieren läßt.

2. Verfahren gemäß Anspruch 1, **dadurch gekennzeichnet, daß** man als Trägerkatalysator einen Trägerkatalysator bestehend aus metallischem Silber auf einem inerten Träger verwendet.

3. Verfahren gemäß Anspruch 1, **dadurch gekennzeichnet, daß** die Trägerkatalysatoren der Reaktionsschritte c) und d) in unmittelbarem Kontakt stehen.

4. Verfahren gemäß Anspruch 1, **dadurch gekennzeichnet, daß** man in den Reaktionsschritten c) und d) als Trägerkatalysator den gleichen Silber-Trägerkatalysator verwendet und die Katalysatoren der Reaktionsschritte c) und d) in unmittelbarem Kontakt stehen.

5. Verfahren gemäß Anspruch 1, **dadurch gekennzeichnet, daß** man in den Reaktionsschritten c) und d) einen Silber-Trägerkatalysator verwendet, der aus Kugeln eines inerten Trägermaterials bestehen, die mit 0,1 bis 20 Gew.-%, bezogen auf die Menge des Trägers, einer Schicht aus metallischem Silber in Form einer glatten, abriebfesten Schale beschichtet sind, und wobei für den größten Durchmesser d der beschichteten Trägerkatalysatoren ein Verhältnis zu dem Innendurchmesser D der Reaktionsrohre von d/D = 0,05 bis 0,3, vorzugsweise 0,1 bis 0,2, gilt.

6. Verfahren gemäß Anspruch 1, **dadurch gekennzeichnet, daß** die mindestens 0,5 cm dicke Schicht gemäß Reaktionsschritt c) den Querschnitt des gesamten Reaktors einnimmt.

7. Verfahren gemäß Anspruch 1, **dadurch gekennzeichnet, daß** man im Reaktionsschritt c) den Sauerstoff enthaltenden Alkoholdampf zunächst durch eine 0,6 bis 5 cm dicke Schicht eines Silber-Trägerkatalysators leitet.

8. Verfahren gemäß Anspruch 1, **dadurch gekennzeichnet, daß** man im Reaktionsschritt d) den Sauerstoff enthaltenden Alkoholdampf in einer Vielzahl von Reaktionsrohren mit einem Innendurchmesser von 1 bis 2 cm und einer Länge von 35 bis 60 cm zu dem entsprechenden Aldehyd reagieren läßt.

9. Verfahren gemäß Anspruch 1, **dadurch gekennzeichnet, daß** man zur Herstellung von 3-Methyl-2-buten-1-al
a) 3-Methyl-2-buten-1-ol und/oder 3-Methyl-3-buten-1-ol verdampft,
b) den 3-Methyl-2-buten-1-ol und/oder 3-Methyl-3-buten-1-ol enthaltenden Dampf mit einem Sauerstoff-enthaltenden Gas versetzt,
c) den erhaltenen Sauerstoff-enthaltenden Dampf aus 3-Methyl-2-buten-1-ol und/oder 3-Methyl-3-buten-1-ol bei Temperaturen kurz oberhalb des Taupunktes durch eine 0,5 bis 4 cm dicke Silber-Trägerkatalysator-Schicht leitet,
d) den Sauerstoff-enthaltenden Dampf aus 3-Methyl-2-buten-1-ol und/oder 3-Methyl-3-buten-1-ol in einer der gewünschten Kapazität entsprechenden Anzahl von mit einem Silber-Trägerkatalysator gefüllten Reaktionsrohren mit einem Innendurchmesser von 1 bis 2 cm und einer Länge von 35 bis 60 cm zu einem Gemisch aus 3-Methyl-2-buten-1-al und 3-Methyl-3-buten-1-al reagieren läßt und
e) das in dem erhaltenen Gemisch enthaltene 3-Methyl-3-buten-1-al in an sich bekannter Weise zu 3-Methyl-2-buten-1-al isomerisiert.

## Claims

1. A process for continuous industrial production of aldehydes of the general formula I 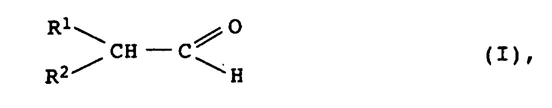 where (isoprenal) R¹ is hydrogen and R² is 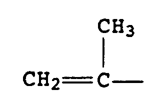 or where (prenal) R¹ and R² are together 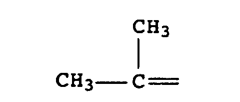 by oxidative dehydrogenation of the corresponding alcohols with an oxygen-comprising gas over a supported catalyst consisting of copper, silver and/or gold on an inert support in a tube bundle reactor, rapid cooling of the reaction gases and removal of the aldehydes from the resulting condensate while recycling the unconverted alcohols, which comprises
a) vaporizing 3-alkylbuten-1-ols of the general formula II 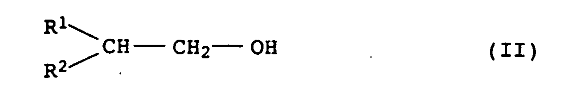
b) admixing the alcohol vapor with an oxygen-comprising gas,
c) initially passing the resulting oxygen-comprising alcohol vapor at above the dew point of the alcohol but below the commencement temperature of the reaction through a layer of one of the abovementioned supported catalysts which is at least 0.5 cm in thickness and only then
d) reacting the oxygen-comprising alcohol vapor at from 300 to 600°C in a sufficient number, for the desired capacity, of parallel reaction tubes surrounded by a fluidic heat transfer medium, packed with the supported catalyst and having an internal diameter D of from about 0.5 to 3 cm and a length of at least 5 cm, to form the corresponding aldehyde.

2. A process as claimed in claim 1, wherein the supported catalyst used is a supported catalyst consisting of metallic silver on an inert support.

3. A process as claimed in claim 1, wherein the supported catalysts of reaction steps c) and d) are in direct contact.

4. A process as claimed in claim 1, wherein reaction steps c) and d) are carried out with the same supported silver catalyst and the catalysts of reaction steps c) and d) are in direct contact.

5. A process as claimed in claim 1, wherein reaction steps c) and d) are carried out with a supported silver catalyst which consists of spheres of an inert support material which have been coated with from 0.1 to 20% by weight, based on the amount of the support, of a layer of metallic silver in the form of a smooth, attrition-resistant shell, the largest diameter d of the coated supported catalyst spheres being subject to a relation with the internal diameter D of the reaction tubes of d/D = 0.05 - 0.3, preferably 0.1 - 0.2.

6. A process as claimed in claim 1, wherein the layer of reaction step c) which is at least 0.5 cm in thickness occupies the cross section of the entire reactor.

7. A process as claimed in claim 1, wherein the layer of reaction step c) through which the oxygen-comprising alcohol vapor is passed initially is a layer of a supported silver catalyst from 0.6 to 5 cm in thickness.

8. A process as claimed in claim 1, wherein the reaction of the oxygen-comprising alcohol vapor in reaction step d) to form the corresponding aldehyde is carried out in a multiplicity of reaction tubes having an internal diameter of from 1 to 2 cm and a length of from 35 to 60 cm.

9. A process as claimed in claim 1, wherein 3-methyl-2-buten-1-al is prepared by
a) vaporizing 3-methyl-2-buten-1-ol and/or 3-methyl-3-buten-1-ol,
b) admixing the 3-methyl-2-buten-1-ol and/or 3-methyl-3-buten-1-ol vapor with an oxygen-comprising gas,
c) passing the resulting oxygen-comprising vapor of 3-methyl-2-buten-1-ol and/or 3-methyl-3-buten-1-ol at just above the dew point through a layer of a supported silver catalyst which is from 0.5 to 4 cm in thickness,
d) reacting the oxygen-comprising vapor of 3-methyl-2-buten-1-ol and/or 3-methyl-3-buten-1-ol in a sufficient number, for the desired capacity, of reaction tubes which are packed with a supported silver catalyst and have an internal diameter of from 1 to 2 cm and a length of from 35 to 60 cm to form a mixture of 3-methyl-2-buten-1-al and 3-methyl-3-buten-1-al, and
e) isomerizing 3-methyl-3-buten-1-al present in the resulting mixture into 3-methyl-2-buten-1-al in a conventional manner.

## Revendications

1. Procédé de fabrication en continu des aldéhydes de formule générale I 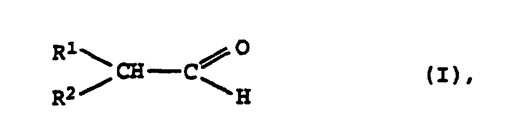 dans laquelle R¹ représente un atome d'hydrogène et R² représente le résidu 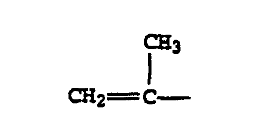 (isoprénal), ou bien R¹ et R² représentent conjointement le résidu 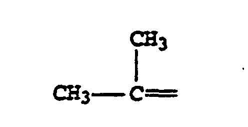 (prénal),
consistant à réaliser une déshydrogénation oxygénée des alcools correspondants avec un gaz contenant de l'oxygène sur un catalyseur supporté qui est constitué de cuivre, d'argent et/ou d'or sur un support inerte dans un réacteur à faisceaux tubulaires, à refroidir rapidement les gaz réactionnels et à séparer les aldéhydes du condensat et à recycler les alcools n'ayant pas réagi, **caractérisé en ce qu'**il comprend les étapes consistant:
a) à vaporiser des 3-alkyl-butèn-1-ol de formule générale II 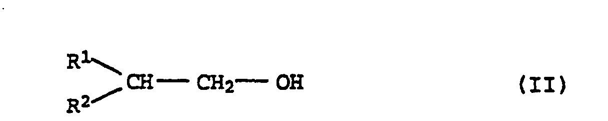
b) à ajouter à l'alcool vaporisé un gaz contenant de l'oxygène,
c) à conduire la vapeur d'alcool obtenue et contenant de l'oxygène au travers d'une couche d'un catalyseur supporté tel que nommé ci-dessus, d'une épaisseur d'au moins 0,5 cm, d'abord à une température supérieure au point de condensation de l'alcool et inférieure à la température initiale de la réaction, et seulement après
d) à mettre à réagir la vapeur d'alcool contenant de l'oxygène dans des tubes de réacteur ayant un diamètre intérieur D d'environ 0,5 cm à 3 cm et une longueur d'au moins 5 cm, en nombre correspondant au besoin, qui sont arrangés de manière parallèle, pourvu d'un écoulement de contournement, d'un caloporteur fluide, et qui contiennent le catalyseur supporté, à une température comprise entre 300 °C et 600 °C, pour obtenir l'aldéhyde correspondant.

2. Procédé selon la revendication 1, **caractérisé en ce que** l'on met en oeuvre en tant que catalyseur supporté, un catalyseur supporté qui est constitué d'argent pur sur un support inerte.

3. Procédé selon la revendication 1, **caractérisé en ce que** les catalyseurs supportés des étapes réactionnelles c) et d) sont en contact direct.

4. Procédé selon la revendication 1, **caractérisé en ce que** l'on met en oeuvre en tant que catalyseur supporté dans les étapes réactionnelles c) et d) le même catalyseur à base d'argent supporté et que les catalyseurs supportés des étapes réactionnelles c) et d) sont en contact direct.

5. Procédé selon la revendication 1, **caractérisé en ce que** l'on met en oeuvre dans les étapes réactionnelles c) et d) un catalyseur à base d'argent supporté, qui est constitué d'un matériau support inerte sous forme de billes, qui sont recouvertes d'une couche d'argent pur à raison de 0,1 % à 20 % en poids par rapport au poids du support, sous forme d'enveloppe lisse et résistante à l'usure, où la relation d/D entre le diamètre maximum des catalyseurs revêtus d et le diamètre intérieur des tubes de réacteur D est comprise entre 0,05 et 0,3, de préférence entre 0,1 et 0,2.

6. Procédé selon la revendication 1, **caractérisé en ce que** la couche d'une épaisseur de 0,5 cm au minimum selon l'étape de réaction c) occupe toute la coupe transversale de tout le réacteur.

7. Procédé selon la revendication 1, **caractérisé en ce que** l'on conduit dans l'étape de réaction c) la vapeur d'alcool contenant de l'oxygène en premier lieu au travers d'une couche d'un catalyseur à base d'argent et supporté.

8. Procédé selon la revendication 1, **caractérisé en ce que** l'on met à réagir dans l'étape de réaction d) la vapeur d'alcool contenant de l'oxygène dans un grand nombre de tubes de réacteur ayant un diamètre intérieur compris entre 1 cm et 2 cm et une longueur comprise entre 35 cm et 60 cm pour obtenir l'aldéhyde correspondant.

9. Procédé selon la revendication 1, **caractérisé en ce que** la préparation de 3-méthyl-2-butèn-1-al comprend les étapes consistant:
a) à vaporiser du 3-méthyl-2-butèn-1-ol et/ou du 3-méthyl-3-butèn-1-ol,
b) à ajouter un gaz contenant de l'oxygène à la vapeur contenant du 3-méthyl-2-butèn-1-ol et/ou du 3-méthyl-3-butèn-1-ol,
c) à conduire la vapeur contenant de l'oxygène obtenue et constituée de 3-méthyl-2-butèn-1-ol et/ou de 3-méthyl-3-butèn-1-ol à une température juste au dessus du point de condensation à travers une couche d'un catalyseur à base d'argent supporté d'une épaisseur comprise entre 0,5 cm à 4 cm,
d) à mettre à réagir la vapeur contenant de oxygène et constituée de 3-méthyl-2-butèn-1-ol et/ou de 3-méthyl-3-butèn-1-ol dans des tubes de réacteur ayant un diamètre interne compris entre 1 cm et 2 cm et une longueur comprise entre 35 cm et 60 cm, en nombre correspondant à la demande, chargés d'un catalyseur à base d'argent supporté, pour obtenir un mélange de 3-méthyl-2-butèn-1-al et de 3-méthyl-3-butèn-1-al,
e) à réaliser de manière généralement connue une isomérisation du 3-méthyl-3-butèn-1-al contenu dans le mélange obtenu pour obtenir du 3-méthyl-2-butèn-1-al.
